Europäisches Patentamt

(19) European Patent Office

Office européen des brevets

(11) Publication number: **0 123 661**
**B1**

(12) **EUROPEAN PATENT SPECIFICATION**

(45) Date of publication of patent specification: **27.05.87**

(21) Application number: **84850091.4**

(22) Date of filing: **22.03.84**

(51) Int. Cl.⁴: **A 61 F 5/453**

(54) Incontinence protection for men.

(30) Priority: **24.03.83 SE 8301646**

(43) Date of publication of application:
**31.10.84 Bulletin 84/44**

(45) Publication of the grant of the patent:
**27.05.87 Bulletin 87/22**

(84) Designated Contracting States:
**AT BE CH DE FR GB IT LI LU NL SE**

(56) References cited:
**WO-A-81/03609**
**CH-A- 553 694**
**US-A-2 933 085**
**US-A-3 403 410**
**US-A-3 579 652**

(73) Proprietor: **Leissner, Karl-Henrik**
**Västerbergsgatan 13**
**S-431 39 Möindal (SE)**

(72) Inventor: **Leissner, Karl-Henrik**
**Västerbergsgatan 13**
**S-431 39 Möindal (SE)**

(74) Representative: **Olsson, Gunnar et al**
**Nobel Corporate Services Patents and**
**Trademarks**
**S-691 84 Karlskoga (SE)**

Courier Press, Leamington Spa, England.

## Description

Background

The present invention relates to an incontinence protection for men including means for collecting urine, which is intended to be attached on the penis.

Incontinence or urine, i.e. involuntary urine leakage, is a social and physical problem for those affected.

The participation of the society in this type of handicap is reflected in the charge-free administration of protection means allowed by official institutes for handicapped.

The incontinent person, to whom no surgical or medical cure exists, is left to hide the handicap as far as possible. Hereby a first purpose is to avoid wetting of clothing and furniture, a second purpose to avoid urine odour.

Urine incontinence in its most serious form is a continuous unvoluntary urine leakage. Between this condition and the normal continent condition there are numerous intermediate types and the occasional drop incontinence is the most common type. The problem with drop incontinence is much greater than normally considered since this handicap is so embarrasing for the affected individual that it seldom is discussed.

Urine incontinence affects both men and women and the number of urine incontinent persons in the population grows and becomes more serious with increasing age.

Urine incontinence is five times more common among women than among men. At an age of 16 to 54 years 8.5 percent of the women and 1.6 percent of the men are urine incontinent. The corresponding figures above 65 years of age are 11.6 for women and 6.9 for men. This means that about one man out of 14 has a more or less pronounced involuntary urine leakage.

The treatment, which can be either medical or surgical, is mainly directed to those with permanent urine leakage. Other individuals with more or less pronounced drop incontinence are often treated with different kinds of urine collecting devices.

Hereby it has been estimated that about 5 percent of all men between the ages of 45 and 85 years are periodically incontinent, i.e. drop incontinent.

This means that one man out of 20 at some occasion requires an incontinence protection for men.

Commercially available are sanitary towels for men adapted to collect urine drops. These bag shaped protections are not safe to wear due to their open construction and cannot after use be sealed odourlessly.

The bar security depends on the risk for unintended release of the urethra from the bag due to the open construction. No sealing means are present.

Different kinds of urine collecting devices in the form of bags adapted to be attached to the penis are also previously known, see for instance WO—A—81/03609. According to this document an elastic tubular envelope is attached to the penis by an adhesive. A device, which can have an oval form, is used to facilitate putting the envelope onto the penis.

The present invention

The main object of the present invention is to avoid the above problems and provide an incontinence protection with improved security against leakage as well as involuntary release. Other objects are to simplify handling, among others attaching, releasing and sealing of the used protection. A further object is to provide a protection which gives as little discomfort as possible.

These objects are reached by the features mentioned in the claims.

By providing the incontinence protection with a clip ring of a generally oval form and by designing this of an elastically resilient material, so that the size of the oval in the short axis direction can be enlarged by compression of the oval in the direction of the oval long axis, the oval at compression will have a more circular cross-section suitable for threading on the penis and an effective fixation after removal of the compressing force will result from the spring-back effect of the resilient material. In order to adapt the pressure exerted in the returned position the clip ring is designed such that the oval shape is retained in the unstressed position, which is most easily obtained by manufacturing the ring with this base form. It is preferred that the parts of the oval meeting at the long axis are extended in the direction of the long axis so that they form tips at the points of connection since these extensions in the long axis direction contributes to a greater flexibility in the short axis direction, without unsuitable increase of pressure or distortion. The size of the clip ring in the long axis direction can for example be between 5 and 15 cm, somewhat depending on the size of the urine collector attached to the clip ring. Even if the size in the short axis direction has a substantial flexibility, this size should to some extent be adapted to the individual and preferably incontinence protections with different sizes in this direction are manufactured, for example in sizes between 2 and 4 cm. The clip ring can be manufactured in different materials such as plastics, rubber or metal, plastics being preferred. Optionally the ring can be manufactured in composite materials, for example with a special built in material giving increased and for the purpose adapted spring effect. Optionally the interior side of the ring can also be provided with a soft and/or a frictional material.

The clip ring shall be attached to a collecting device for urine, which in its simplest form can be a towel or other type of absorbing material. It is, however, preferred that the device is in the form of a bag at the opening of which the clip ring is applied. The bag can be of paper, natural fibres, napkin or another absorbing material but it is preferred that the bag includes an impermeable material, preferably a plastic, possibly containing an absorbing material. With bags of impermeable materials bad odour can be prevented if the clip ring is attached to the entire periphery of the bag opening whereby air exchange at the opening can be fully avoided in use. The bag need not be permanently attached to the ring but can be threaded through the ring and held in place by the applied pressure between the ring and the penis. It is preferred, however, that the ring is fixed to the bag by glueing, fusing or another suitable way.

The security against unvoluntary release of the incontinence protection can be improved by providing the front side of the ring, or better the urine collector, with a friction surface of for example foamed plastic, teasel bands or pressure sensitive tape, whereby the leg clothes will participate in keeping the protection in place. The reverse side of the protection can with advantage be provided with a surface lenient to the skin, for example foamed plastic or another material supporting air circulation and preventing "burning" effects of for example plastics against the skin.

For bag shaped collecting devices it is further preferred that sealing means are arranged at the bag opening allowing sealing of the opening after use. Such a sealing possibility can most easily be arranged by providing one side of the bag with a flap which can be folded over the clip ring and attached to the other side of the bag. It is hereby suitable to give the flap self-sealing properties. If the flap is arranged on the front side of the bag and is provided with a friction surface this surface can act, as said above, as friction increasing means against the clothing during use of the protection with the flap folded down while after use the flap can be folded over and pressed against the other surface of the bag whereby the friction surface can act as a seal against this surface, especially if the reverse side of the bag is provided with foamed plastic or a similar material as aforementioned. With this construction the clip ring can be made cooperative in a safe sealing if it is slightly compressed at the sealing so that its resilient force exerts a positive pressure on the sealing surface.

Description of the drawing

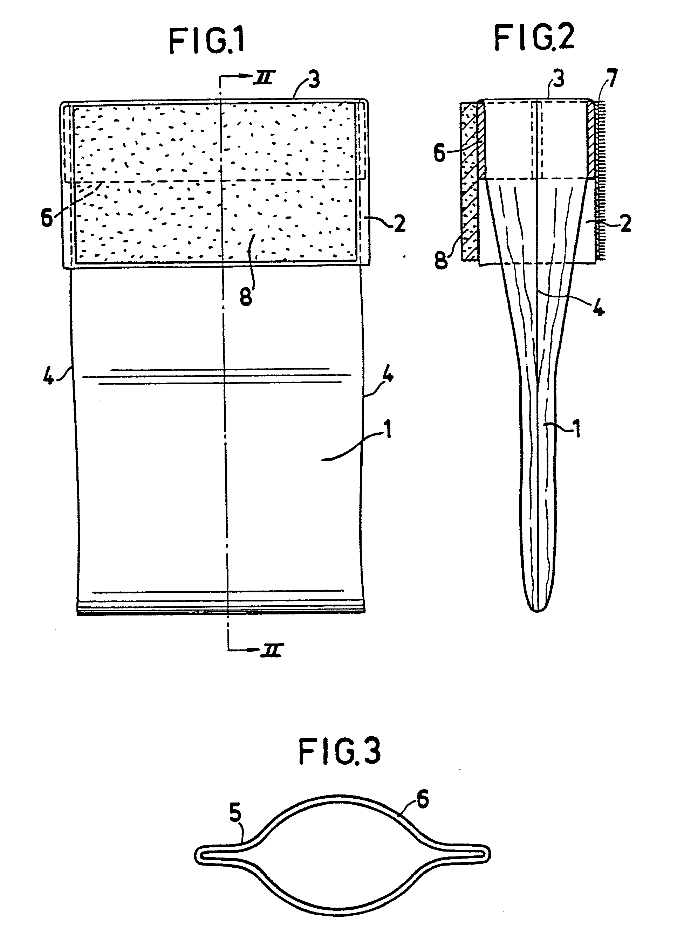

The drawing shows a preferred embodiment of the invention in front view in Figure 1 and side view in Figure 2 as well as the clip ring in a view from above in Figure 3.

With 1 is indicated a urine collecting device in the form of a plastic bag, which in this particular embodiment has a width of about 10 cm and a height of about 15 cm. The upper part of the bag 1 is threaded through the ring 6 and is folded back over the exterior side of the ring to form a surrounding flap 2 extending about 6 cm down from the opening 3 at the upper edge of the ring 6. The flap 2 is provided on the intended front facing surface with a friction increasing layer 7 of for example teasle type or another anti-slip surface. The intended rear facing side of the flap is provided with a layer 8 of for example plastic foam or paper. For best rigidity the side edges of the bag may initially be joined together along the flap 2 during insertion through the ring and can then be torn apart for example in connection with the folding of the front part of the flap over the ring, which should be compressed at this stage, and in connection with the folding of the rear part of the flap over the other side of the ring. As is evident from the figure the cross-section of the ring 6 has a portion 5 where the curvature changes direction so that tips are formed at the point of connection between the halves of the oval at the end points of the long axis. The ring has a height of about 1.5 cm and a generally uniform cross-section over the height. The exterior of the bag 1 may be fixed to the interior of the ring 6 by glueing or fusing.

## Claims

1. An incontinence protection for men including means (1) for collecting urine adapted to be attached to the penis characterized in that said means (1) for collecting urine is equipped with a clip ring (6) of elastically resilient material of a generally oval form with a short and a long symmetry axis, whereby the size of the oval in the short axis direction can be enlarged by compression of the clip ring (6) in the long axis direction and that the clip ring (6) is unstressed condition retains the generally oval form.

2. Protection according to claim 1, characterized in that the form of the clip ring (6) is extended into tips (5) in the direction of the long axis.

3. Protection according to claim 1, characterized in that the clip ring (6) interior is provided with friction improving means.

4. Protection according to claim 1, characterized in that the means (1) for collecting urine includes a receptacle at the opening (3) of which the clip ring (6) is arranged.

5. Protection according to claim 4, characterized in that the receptacle can be sealed by folding a flap (2) arranged at the opening (3) over the opening and attaching it to the other side of the receptacle.

6. Protection according to claim 5, characterized in that the flap (2) is provided with a friction material (7).

7. Protection according to claim 5, characterized in that the exterior surface remote from the flap (2) of the receptacle opening (3) is provided with a porous material (8).

## Patentansprüche

1. Inkontinenzschutz für Männer mit einer Urinsammelvorrichtung, die am Penis befestigt werden kann, da durch gekennzeichnet dass die Urinsammelvorrichtung (1) mit einem Klemmring (6) aus elastischem Material von im wesentlichen ovaler Form mit einer kurzen und einer langen Symmetrieachse versehen ist, wodurch die Grösse des Ovals der kurzen Achsenrichtung durch Kompression des Klemmrings (6) in der Richtung der langen Achse verlängert werden kann, und dass der Klemmring (6) in ungespanntem Zustand die im wesentlichen ovale Form annimmt.

2. Inkontinenzschutz nach Anspruch 1, dadurch gekennzeichnet, dass die Form des Klemmrings (6) in der Richtung der langen Achse sich in Spitzen (5) verlängert.

3. Inkontinenzschutz nach Anspruch 1, dadurch gekennzeichnet, dass der Klemmring (6) an seinem Inneren mit einer Vorrichtung zur Erhöhung der Reibung versehen ist.

4. Inkontinenzschutz nach Anspruch 1, dadurch gekennzeichnet, dass die Urinsammelvorrichtung (1) ein Aufnehmbehältnis aufweist, an dessen Öffnung (3) der Klemmring (6) angeordnet ist.

5. Inkontinenzschutz nach Anspruch 4, dadurch gekennzeichnet, dass das Aufnahmebehältnis abgedichtet werden kann durch Falten einer Klappe (2) an der Öffnung (3) über die Öffnung und Befestigen an der anderen Seite des Aufnahmebehältnisses.

6. Inkontinenzschutz nach Anspruch 5, dadurch gekennzeichnet, dass die Klappe (2) mit einem Friktionsmaterial (7) versehen ist.

7. Inkontinenzschutz nach Anspruch 5, dadurch gekennzeichnet, dass die Aussenfläche gegenüber der Klappe (2) der Öffnung (3) des Aufnahmebehältnisses mit einem porösen Material (8) versehen ist.

**Revendications**

1. Protection contre l'incontinence urinaire masculine, comprenent un moyen (1) pour collecter l'urine et destinée à être fixée sur le pénis, appareil caractérisé en ce que ledit moyen (1) pour collecter l'urine est équipé d'un anneau (6) de serrage en une matière élastique de forme générale ovale présentant un petit axe et un long axe de symétrie, de sorte que la dimension de l'ovale peut être augmentée dans la direction du petit axe par compression de l'anneau (6) de serrage dans la direction du grand axs et que, à l'état non soumis à des efforts de contrainte, l'anneau (6) de serrage conserve sa forme généralement ovale.

2. Protection selon la revendication 1, caractérisée en ce que la forme de l'anneau (6) de serrage est prolongée en des pointes (5) dans la direction du grand axe.

3. Protection selon la revendication 1, caractérisée en ce que l'intérieur de l'anneau (6) de serrage est muni d'un moyen augmentant le frottement.

4. Protection selon la revendication 1, caractérisée en ce que le moyen (1) pour collecter l'urine comprend un réceptacle à l'ouverture (3) duquel l'anneau (6) de serrage est fixé.

5. Protection selon la revendication 4, caractérisée en ce que le réceptacle peut être fermé de façon étanche par pliage d'un rabat (2), disposé à l'ouverture (3) par-dessus l'ouverture et fixation de ce rabat sur l'autre côté du réceptacle.

6. Protection selon la revendication 5, caractérisée en ce que le rabat (2) comporte une matière (7) de friction.

7. Protection selon la revendication 5, caractérisée en ce que la surface extérieure, éloingée du rabat (2) de l'ouverture (3) du réceptacle, est équipée d'une matière (8) poreuse.

## FIG.1

## FIG.2

## FIG.3